# EUROPÄISCHE PATENTANMELDUNG

(11) **EP 1 997 526 A1**
(43) Veröffentlichungstag der Anmeldung: **03.12.2008**
(21) Anmeldenummer: 08157199.4
(22) Anmeldetag: 29.05.2008
(51) Int. Cl.: A61M 25/00, A61M 25/10

(54) **Mehrwege-Katheter**

(30) Priorität: 01.06.2007 DE 202007007713 U
(71) Anmelder: Urovision Gesellschaft für Medizinischen Technologie Transfer mbH, 83043 Bad Aibling (DE)
(72) Erfinder: Skrezek, Dr. Christian, 38440 wolsfburg (DE)
(74) Vertreter: Hofstetter, Alfons J.

(57) **Zusammenfassung**

Die vorliegende Erfindung betrifft einen Mehrwege-Katheter (10) zum Einführen in die Harnröhre und die Harnblase bestehend aus einem Katheterkörper (12) und mindestens zwei innerhalb des Katheterkörpers (12) angeordneten, koaxial zum Katheterkörper (12) verlaufenden Kanälen (14, 16), wobei der erste Kanal (14) zur Befüllung und Spülung der Harnblase mit einer Flüssigkeit dient und in mindestens einer ersten Austrittsöffnung (22) an einem distalen, körpernahen Ende (24) des Katheters (10) endet und der zweite Kanal (16) zur Befüllung eines am körpernahen Ende (24) des Katheters (10) ausgebildeten Ballons (26) ausgebildet ist. Dabei ist innerhalb des Katheterkörpers (12) mindestens ein dritter Kanal (18) zur Applikation von mindestens einem Medikament koaxial zum Katheterkörper (12) ausgebildet, wobei der dritte Kanal (18) mindestens eine erste Austrittsöffnung (28) aufweist und die erste Austrittsöffnung (28) von dem körpernahen Ende (24) in Richtung eines körperfernen Endes (30) des Katheters (10) versetzt angeordnet ist, derart, dass die erste Austrittsöffnung (28) nach Einführen des Katheters (10) in die Harnröhre und die Harnblase im Bereich der Harnröhre (H) zu liegen kommt.

## Beschreibung

Die vorliegende Erfindung betrifft einen Mehrwege-Katheter zum Einführen in die Harnröhre und die Harnblase bestehend aus einem Katheterkörper und mindestens zwei innerhalb des Katheterkörpers angeordneten, koaxial zum Katheterkörper verlaufenden Kanälen, wobei der erste Kanal zur Befüllung und Spülung der Harnblase mit einer Flüssigkeit dient und in mindestens einer ersten Austrittsöffnung an einem distalen, körpernahen Ende des Katheters endet und der zweite Kanal zur Befüllung eines am körpernahen Ende des Katheters ausgebildeten Ballons ausgebildet ist.

Derartige Mehrwege-Katheter sind bekannt. Es handelt sich dabei insbesondere um transurethale Blasenkatheter die über die Harnröhre in die Blase eines Menschen eingebracht werden. Dabei kann es sich um so genannte Einmal- oder Dauerkatheter handeln. Dauerkatheter werden dabei durch den am körpernahen Ende des Katheters ausgebildeten Ballon in der Blase gehalten und fixiert. Grundsätzlich können bei den genannten Dauerkathetern 2-Wege- und 3-Wege-Katheter unterschieden werden. Bei 2-Wege-Kathetern dienen ein Kanal zur Ableitung des Harns aus der Blase und der zweite Kanal zur Befüllung des genannten Ballons. Bei 3-Wege-Kathetern ist noch ein dritter Kanal ausgebildet, der zur Befüllung und Spülung der Harnblase dient.

Des Weiteren sind zum Beispiel aus der DE 203 08 756 U 1 mehrlumige Urodynamik-Katheter zur Aufzeichnung des Drucks innerhalb der Harnblase und des Harnröhrendrucks bekannt. Nachteilig an den bekannten Mehrwege-Kathetern ist jedoch, dass diese zwar zur Verwendung als transurethraler Dauerkatheter geeignet sind, weitere Anwendungsmöglichkeiten wie zum Beispiel die gezielte Applikation von Medikamenten im Bereich der Harnröhre aber nicht möglich sind.

Es ist daher Aufgabe der vorliegenden Erfindung einen Mehrwege-Katheter der eingangs genannten Art bereitzustellen, welcher neben der Verwendung als transurethraler Dauerkatheter auch für die gezielte Applikation von Medikamenten im Bereich der Harnröhre geeignet ist.

Gelöst wird diese Aufgabe durch einen Mehrwege-Katheter mit den Merkmalen des Anspruchs 1.

Vorteilhafte Ausgestaltungen sind in den Unteransprüchen beschrieben.

Ein erfindungsgemäßer Mehrwege-Katheter zum Einführen in die Harnröhre und die Harnblase eines Menschen besteht aus einem Katheterkörper und mindestens zwei innerhalb des Katheterkörpers angeordneten, koaxial zum Katheterkörper verlaufenden Kanälen. Der erste Kanal dient dabei zur Befüllung und Spülung der Harnblase mit einer Flüssigkeit und endet in mindestens einer Austrittsöffnung an einem distalen, körpernahen Ende des Katheters. Der zweite Kanal ist zur Befüllung eines am körpernahen Ende des Katheters ausgebildeten Ballons ausgebildet. Gemäß der Erfindung ist innerhalb des Katheterkörpers mindestens ein dritter Kanal zur Applikation von mindestens einem Medikament koaxial zum Katheterkörper ausgebildet, wobei der dritte Kanal mindestens eine erste Austrittsöffnung aufweist und die erste Austrittsöffnung von dem körpernahen Ende in Richtung eines körperfernen Endes des Katheters versetzt angeordnet ist, derart, dass die erste Austrittsöffnung nach Einführen des Katheters in die Harnröhre und die Harnblase im Bereich der Harnröhre zu liegen kommt. Eine derartige erfindungsgemäße Ausgestaltung des dritten Kanals ermöglicht die gezielte Applikation von Medikamenten in dem Bereich der Harnröhre über den dritten Kanal des Katheters. Damit ist es vorteilhafterweise möglich, dass neben der üblichen Harnableitung, die ebenfalls über den ersten Kanal erfolgen kann, und der Spülung der Blase nunmehr zusätzlich eine gezielte medikamentöse Behandlung erkrankter Bereiche der Harnröhre des Patienten durchgeführt werden kann.

In einer weiteren vorteilhaften Ausgestaltung des erfindungsgemäßen Mehrwege-Katheters weist der dritte Kanal mindestens eine weitere, zweite Austrittsöffnung auf, wobei die zweite Austrittsöffnung zwischen der ersten Austrittsöffnung und dem am körpernahen Ende des Katheters ausgebildeten Ballon angeordnet ist, derart, dass die zweite Austrittsöffnung nach Einführen des Katheters in die Harnröhre und die Harnblase im Bereich der Prostata zu liegen kommt. Mit dieser erfindungsgemäßen Ausführungsform ist es möglich, dass neben der Behandlung erkrankter Harnröhrenbereiche auch der von der Prostata umgebene Bereich der Harnröhre medikamentös über den dritten Kanal behandelt werden kann. Zum Beispiel können über den dritten Kanal entzündungshemmende Flüssigkeiten appliziert werden.

In einer weiteren vorteilhaften Ausgestaltung des erfindungsgemäßen Mehrwege-Katheters ist innerhalb des Katheterkörpers mindestens ein vierter Kanal zur Applikation von mindestens einem Medikament koaxial zum Katheterkörper ausgebildet, wobei der vierte Kanal mindestens eine Austrittsöffnung aufweist und die Austrittsöffnung zwischen der ersten Austrittsöffnung und dem am distalen, körpernahen Ende des Katheters ausgebildeten Ballons angeordnet ist, derart, dass die Austrittsöffnung nach Einführen des Katheters in die Harnröhre und die Harnblase im Bereich der Prostata zu liegen kommt. Durch die Ausbildung eines weiteren, vierten Kanals ist es vorteilhafterweise möglich, dass verschiedene Medikamente zum gleichen Zeitpunkt oder nacheinander über den dritten und vierten Kanal appliziert werden können. Diese Medikamente können gezielt auf einerseits den Bereich der Harnröhre und andererseits den Bereich der Prostata getrennt voneinander appliziert werden.

In einer weiteren vorteilhaften Ausgestaltung des erfindungsgemäßen Mehrwege-Katheters weist dieser im Bereich des körpernahen Endes mindestens eine röntgendichte Markierung auf. Damit kann vorteilhafterweise die Lage des Katheters innerhalb des Körpers kontrolliert werden. Insbesondere kann überprüft werden, ob die unterschiedlichen Austrittsöffnungen im Bereich der Harnröhre und im Bereich der Prostata zu liegen kommen.

In einer weiteren vorteilhaften Ausgestaltung der Erfindung weisen die Kanäle im Bereich des körperfernen Endes des Katheters jeweils mindestens eine Anschlussvorrichtung auf. Dabei kann der zweite Kanal über die zweite Anschlussvorrichtung und einen daran angeordneten Schlauch mit einer Füllpumpe zur Befüllung des Ballons mit einer entsprechenden Flüssigkeit verbunden sein. An die erste Anschlussvorrichtung können Vorrichtungen zur Befüllung und Spülung der Harnblase mit entsprechenden Flüssigkeiten angeschlossen werden. Die dritte und vierte Anschlussvorrichtung dient jeweils zur Einbringung der Medikamente, die üblicherweise in flüssiger Form vorliegen, in den dritten und/oder vierten Kanal des Mehrwege-Katheters.

In einer weiteren vorteilhaften Ausgestaltung der Erfindung ist am Katheterkörper im Bereich des körperfernen Endes des Katheters ein auf dem Katheterkörper verschiebbarer Konus zum Verschluss der Harnröhre angeordnet. Damit ist gewährleistet, dass die applizierten Medikamente nicht unbeabsichtigt aus der Harnröhre austreten können.

In einer weiteren vorteilhaften Ausgestaltung der Erfindung ist am Katheterkörper im Bereich des körperfernen Endes des Katheters eine Klemmvorrichtung angeordnet. Mit dieser Klemmvorrichtung ist es vorteilhafterweise möglich den Katheter bzw. die jeweiligen Katheterkanäle abzuschließen und somit einen Flüssigkeitsrückfluss aus den entsprechenden Kanälen zu verhindern. Zudem kann die Klemmvorrichtung vorteilhafterweise zur Befestigung des im vorhergehenden beschriebenen verschiebbaren Konus verwendet werden.

Weitere Einzelheiten, Merkmale und Vorteile der Erfindung ergeben sich aus der nachfolgenden Beschreibung zweier in den Figuren dargestellter Ausführungsbeispiele. Es zeigen
- Figur 1: eine schematische Darstellung eines erfindungsgemäßen Mehrwege-Katheters gemäß einer ersten Ausführungsform;
- Figur 2: eine Querschnittsdarstellung des erfindungsgemäßen Mehrwege-Katheters gemäß Figur 1;
- Figur 3: eine schematische Darstellung eines erfindungsgemäßen Mehrwege-Katheters gemäß einer zweiten Ausführungsform; und
- Figur 4: eine Querschnittsdarstellung des erfindungsgemäßen Mehrwege-Katheters gemäß Figur 3.

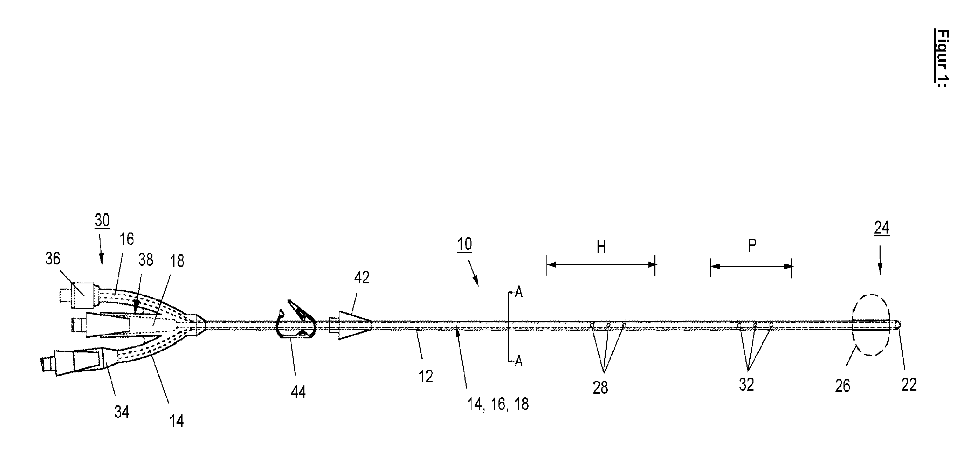

Figur 1 zeigt in einer schematischen Darstellung einen Mehrwege-Katheter 10 zum Einführen in die Harnröhre und die Harnblase eines Menschen. Der Katheter 10 besteht dabei aus einem Katheterkörper 12, wobei innerhalb des Katheterkörpers 12 ein koaxial zum Katheterkörper 12 ein erster Kanal 14 zur Befüllung und Spülung der Harnblase mit einer Flüssigkeit und zur Harnableitung ausgebildet ist. Der erste Kanal 14 weist hierfür mindestens eine Austrittsöffnung 22 an einem distalen, körpernahen Ende 24 des Katheters auf. Des Weiteren ist ein zweiter Kanal 16 zur Befüllung eines am körpernahen Ende 24 des Katheters 10 ausgebildeten Ballons 26 ebenfalls koaxial zum Katheterkörper 12 angeordnet. Der Ballon 26 dient dabei zur Fixierung des Katheters 10 innerhalb der Blase. Des Weiteren erkennt man, dass bei dieser gezeigten ersten Ausführungsform der Katheter 10 einen dritten Kanal 18, der ebenfalls koaxial zum Katheterkörper 12 verläuft aufweist, wobei der dritte Kanal 18 mehrere erste Austrittsöffnungen 28 aufweist und die ersten Austrittsöffnungen 28 von dem körpernahen Ende 24 in Richtung eines körperfernen Endes 30 des Katheters 10 versetzt angeordnet sind. Die Anordnung erfolgt dabei derart, dass die ersten Austrittsöffnungen 28 nach Einführen des Katheters 10 in die Harnröhre und die Harnblase in einem Bereich H der Harnröhre zu liegen kommen. In dem dargestellten Ausführungsbeispiel weist der dritte Kanal 18 zudem zweite Austrittsöffnungen 32 auf, wobei die zweiten Austrittsöffnungen 32 zwischen den ersten Austrittsöffnungen 28 und dem Ballon 26 angeordnet sind, derart, dass die zweiten Austrittsöffnungen 32 nach Einführen des Katheters 10 in die Harnröhre und die Harnblase in einem von der Prostata umgebenen Bereich P der Harnröhre zu liegen kommen. Über die Austrittsöffnungen 28, 32 können an die genannten Bereiche der Harnröhre Medikamente appliziert werden. Des Weiteren erkennt man, dass die ersten Austrittsöffnungen 28 und die zweiten Austrittsöffnungen 32 jeweils hintereinander in den genannten Bereichen angeordnet sind. Weitere, am Umfang des Katheterkörpers 12 ausgebildete Austrittsöffnungen sind möglich. Der Abstand zwischen der dem distalen Ende 24 des Katheters 10 am nächsten liegenden Austrittsöffnung 28 und der körperfernsten zweiten Austrittsöffnung 32 beträgt üblicherweise 60 bis 80 Millimeter. Auch andere Abstände sind denkbar. Der Durchmesser der ersten und zweiten Austrittsöffnungen 28, 32 kann zwischen 1,0 bis 3,0 Millimeter, abhängig von dem Durchmesser des Katheterkörpers 12, betragen. Der Katheter 10 besteht üblicherweise aus Latex, PVC oder Silikon, zudem kann der Katheterkörper 12 zur Erhöhung der Gleitfähigkeit beschichtet sein. Die Auswahl des Kathetermaterials ist im Wesentlichen abhängig von dem Anwendungsbereich des Katheters 10, insbesondere von der Anwendungsdauer.

Des Weiteren erkennt man, dass am Katheterkörper 12 im Bereich des körperfernen Endes 30 des Katheters 10 ein auf dem Katheterkörper 12 verschiebbarer Konus 42 zum Verschluss der Harnröhre angeordnet ist. Zudem ist am Katheterkörper 12, ebenfalls im Bereich des körperfernen Endes 30 des Katheters 10 eine Klemmvorrichtung 44 zum Verschluss des Katheters 10 bzw. der verschiedenen Katheterkanäle angeordnet. Die Klemmvorrichtung 44 kann zudem zur Fixierung des Konus 42 verwendet werden.

Im Bereich des körperfernen Endes 30 des Katheters 10 sind die Kanäle 14, 16, 18 mit jeweils einer Anschlussvorrichtung 34, 36, 38 verbunden. So dient die zweite Anschlussvorrichtung 36 zur Verbindung des zweiten Kanals 16 mit einer Füllpumpe zur Befüllung des Ballons 26. Die erste Anschlussvorrichtung 34 dient zur Applikation entsprechender Spülflüssigkeiten für die Blase und einer entsprechenden Blasenbefüllung sowie gegebenenfalls zur Harnableitung. Die dritte Anschlussvorrichtung 38 dient zur Applikation des jeweiligen Medikaments, welches über den dritten Kanal 18 und die ersten und zweiten Austrittsöffnungen 28, 32 in die unterschiedlichen Bereiche H, P der Harnröhre eingebracht werden sollen.

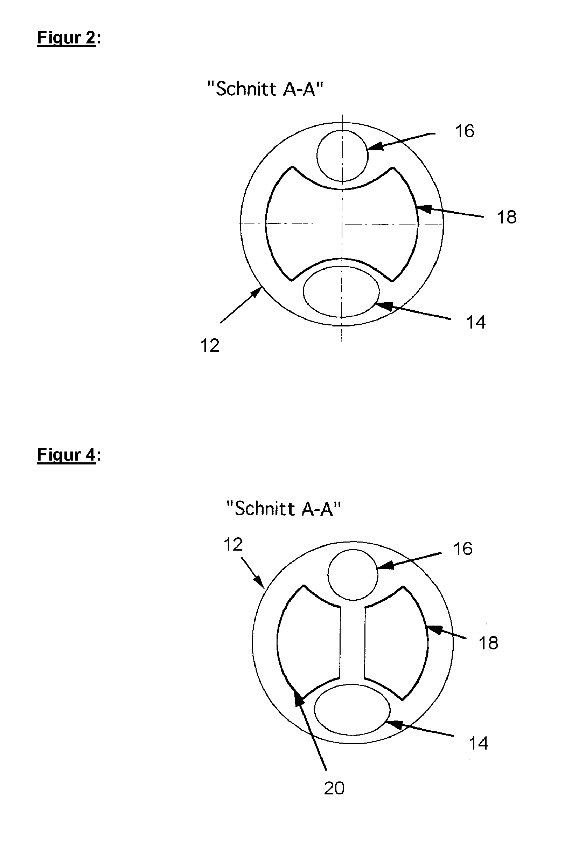

Figur 2 zeigt eine Querschnittsdarstellung des Mehrwege-Katheters gemäß Figur 1. Man erkennt die Ausgestaltung der drei Kanäle 14, 16, 18 innerhalb des Katheterkörpers 12.

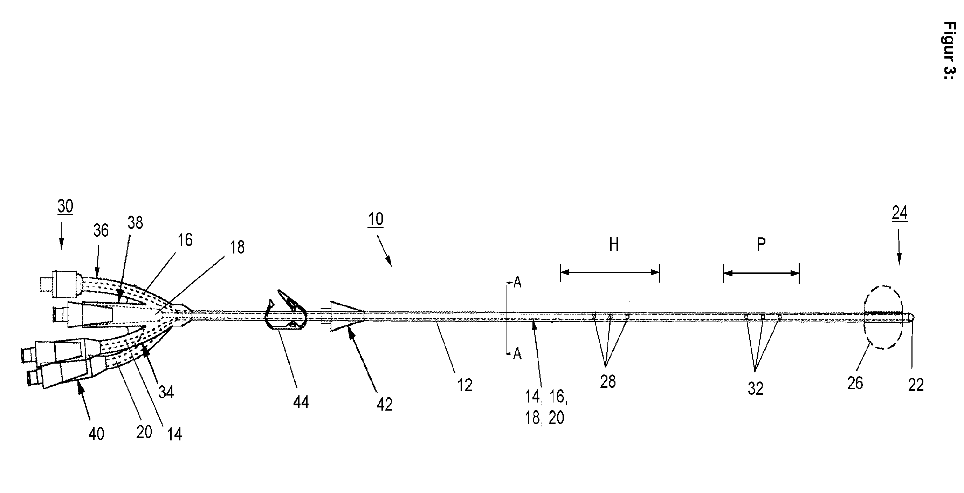

Figur 3 zeigt eine schematische Darstellung eines Mehrwege-Katheters 10 gemäß einer zweiten Ausführungsform. Die Ausgestaltung des in dieser Figur dargestellten Katheters 10 entspricht grundsätzlich dem Aufbau des in den Figuren 1 und 2 beschriebenen Katheters 10. Im Unterschied zu dem dort dargestellten ersten Ausführungsbeispiel ist hier jedoch ein vierter Kanal 20 zur Applikation von mindestens einem Medikament, ebenfalls koaxial zum Katheterkörper 12 ausgebildet. Dabei weist der vierte Kanal 20 mehrere Austrittsöffnungen 32 auf, wobei die Austrittsöffnungen 32 zwischen der ersten Austrittsöffnung 28 des dritten Kanals 18 und dem Ballon 26 angeordnet ist. Deren Anordnung erfolgt derart, dass die Austrittsöffnung 32 nach Einführen des Katheters 10 in die Harnröhre und die Harnblase in dem von der Prostata umgebenen Bereich P der Harnröhre zu liegen kommt. Dies bedeutet, dass in dem zweiten Ausführungsbeispiel der dritte und vierte Kanal 18, 20 jeweils separat ausgebildete Austrittsöffnungen 28, 32 aufweisen. Da der dritte und vierte Kanal 18, 20 zudem jeweils mit einer separat ausgebildeten Anschlussvorrichtung 38, 40 verbunden ist, ist es möglich, über die Anschlussvorrichtungen 38, 40 die jeweiligen Bereiche H, P innerhalb der Harnröhre separat mit unterschiedlichen oder identischen Medikamenten zu behandeln. Die Behandlung kann zudem zeitgleich erfolgen. Bezüglich der weiteren Merkmale des Katheters 10 gemäß dem zweiten Ausführungsbeispiel verweisen wir auf die Beschreibung des Katheters 10 gemäß dem ersten Ausführungsbeispiel.

Figur 4 zeigt eine Querschnittsdarstellung des Mehrwege-Katheters 10 gemäß Figur 3. Man erkennt die Ausbildung von vier unterschiedlichen Kanälen 14, 16, 18, 20 innerhalb des Katheterkörpers 12.

## Patentansprüche

1. Mehrwege-Katheter (10) zum Einführen in die Harnröhre und die Harnblase bestehend aus einem Katheterkörper (12) und mindestens zwei innerhalb des Katheterkörpers (12) angeordneten, koaxial zum Katheterkörper (12) verlaufenden Kanälen (14, 16), wobei der erste Kanal (14) zur Befüllung und Spülung der Harnblase mit einer Flüssigkeit dient und in mindestens einer ersten Austrittsöffnung (22) an einem distalen, körpernahen Ende (24) des Katheters (10) endet und der zweite Kanal (16) zur Befüllung eines am körpernahen Ende (24) des Katheters (10) ausgebildeten Ballons (26) ausgebildet ist,
**dadurch gekennzeichnet,**
**dass** innerhalb des Katheterkörpers (12) mindestens ein dritter Kanal (18) zur Applikation von mindestens einem Medikament koaxial zum Katheterkörper (12) ausgebildet ist, wobei der dritte Kanal (18) mindestens eine erste Austrittsöffnung (28) aufweist und die erste Austrittsöffnung (28) von dem körpernahen Ende (24) in Richtung eines körperfernen Endes (30) des Katheters (10) versetzt angeordnet ist, derart, dass die erste Austrittsöffnung (28) nach Einführen des Katheters (10) in die Harnröhre und die Harnblase im Bereich der Harnröhre (H) zu liegen kommt.

2. Mehrwege-Katheter nach Anspruch 1,
**dadurch gekennzeichnet,**
**dass** der dritte Kanal (18) mindestens eine weitere, zweite Austrittsöffnung (32) aufweist, wobei die zweite Austrittsöffnung (32) zwischen der ersten Austrittsöffnung (28) und dem Ballon (26) angeordnet ist, derart, dass die zweite Austrittsöffnung (32) nach Einführen des Katheters (10) in die Harnröhre und die Harnblase im Bereich der Prostata (P) zu liegen kommt.

3. Mehrwege-Katheter nach Anspruch 1,
**dadurch gekennzeichnet,**
**dass** innerhalb des Katheterkörpers (12) mindestens ein vierter Kanal (20) zur Applikation von mindestens einem Medikament koaxial zum Katheterkörper (12) ausgebildet ist, wobei der vierte Kanal (20) mindestens eine Austrittsöffnung (32) aufweist und die Austrittsöffnung (32) zwischen der ersten Austrittsöffnung (28) und dem Ballon (26) angeordnet ist, derart, dass die Austrittsöffnung (32) nach Einführen des Katheters (10) in die Harnröhre und die Harnblase im Bereich der Prostata (P) zu liegen kommt.

4. Mehrwege-Katheter nach einem der vorhergehenden Ansprüche,
**dadurch gekennzeichnet,**
**dass** der Katheter (10) im Bereich des körpernahen Endes (22) mindestens eine röntgendichte Markierung aufweist.

5. Mehrwege-Katheter nach einem der vorhergehenden Ansprüche,
**dadurch gekennzeichnet,**
**dass** die Kanäle (14, 16, 18, 20) im Bereich des körperfernen Endes (30) des Katheters (10) jeweils mindestens eine Anschlussvorrichtung (34, 36, 38, 40) aufweisen.

6. Mehrwege-Katheter nach Anspruch 5,
**dadurch gekennzeichnet,**
**dass** der zweite Kanal (16) über die zweite Anschlussvorrichtung (36) und einen daran angeordneten Schlauch mit einer Füllpumpe verbunden ist.

7. Mehrwege-Katheter nach einem der vorhergehenden Ansprüche,
**dadurch gekennzeichnet,**
**dass** am Katheterkörper (12) im Bereich des körperfernen Endes (30) des Katheters (10) ein auf dem Katheterkörper (12) verschiebbarer Konus (42) zum Verschluss der Harnröhre angeordnet ist.

8. Mehrwege-Katheter nach einem der vorhergehenden Ansprüche,
**dadurch gekennzeichnet,**
**dass** am Katheterkörper (12) im Bereich des körperfernen Endes (30) des Katheters (10) eine Klemmvorrichtung (44) angeordnet ist.
